# EUROPEAN PATENT APPLICATION

(11) **EP 1 672 079 A1**
(43) Date of publication of application: **21.06.2006**
(21) Application number: 04030123.6
(22) Date of filing: 20.12.2004
(51) Int. Cl.: C12Q 1/68

(54) **Method of diagnosing cancer**

(71) Applicant: Georg-August-Universität Göttingen Stiftung öffentlichen Rechts, 37075 Göttingen (DE)
(72) Inventor: Nayernia, Karim, 73085 Göttingen (DE); Engel, Wolfgang, 37120 Lenglern (DE)
(74) Representative: Wichmann, Hendrik

(57) **Abstract**

The present invention relates to a method of diagnosing cancer other than testicular cancer, the use of an indicator of piwil2 for the diagnosis of cancer, a kit suitable for diagnosing cancer, a method of screening for an inhibitor of piwil2 for use in the treatment or prevention of cancer and the use of an anti-piwil2 antibody, an anti-piwil2 antibody fragment, a microprotein, an antisense polynucleotide or small interfering RNA of a polynucleotide encoding piwil2 for the manufacture of a medicament for treating or preventing cancer.

## Description

The present invention relates to a method of diagnosing cancer other than testicular cancer, the use of an indicator of piwil2 for the diagnosis of cancer, a kit suitable for diagnosing cancer, a method of screening for an inhibitor of piwil2 for use in the treatment or prevention of cancer and the use of an anti-piwil2 antibody, an anti-piwil2 antibody fragment, a microprotein, an antisense polynucleotide or small interfering RNA of a polynucleotide encoding piwil2 for the manufacture of a medicament for treating or preventing cancer.

Cancer is a diverse range of diseases in which abnormal cells grow rapidly and generally spread throughout the body in an uncontrolled manner. These cancerous cells can invade and destroy surrounding tissue and spread to distant parts of the body. Cancer can develop from most types of cells in different parts of the body. Different cancers grow and spread in different ways and at different rates. Cancer is caused by both external factors (such as tobacco, chemicals, radiations and infectious organisms) as well as internal factors (inherited mutations, hormones, immune conditions and mutations that occur from metabolism). Causal factors may act together or in sequence to initiate or promote carcinogenesis. While some cancers share common causes or risk factors, it is believed that most cancers have a unique set of factors causing them to occur. Cancer is in general treated by surgery, radiation, chemotherapy, hormones and immunotherapy.

Cancer has serious impacts on national health. According to the American Cancer Society 556,500 Americans were expected to die of cancer in 2003, i.e. more than 1,500 people a day. Cancer is the second leading cause of death in the US, exceeded only by heart diseases. In the US, 1 of every 4 deaths is from cancer.

Cancer is often difficult to recognize, but numerous studies have shown that early detection of cancer saves lives and increases treatment options. Direct or assisted visual observation is the most widely available examination for the detection of cancer. It is useful in identifying suspicious lesions in the skin, retina, lip, mouth, larynx, external genitalia, and cervix. The second most available detection procedure is palpation to detect lumps, nodules, or tumors in the breast, mouth, salivary glands, thyroid, subcutaneous tissues, anus, rectum, prostate, testes, ovaries, and uterus and enlarged lymph nodes in the neck, axilla, or groin. Internal cancers require procedures and tests such as endoscopy, x-rays, magnetic resonance imaging, or ultrasound.

Additionally, a series of laboratory tests using tumor markers has been developed and employed to detect cancer. Tumor markers are molecules occurring e.g. in blood or tissue that are associated with cancer and whose measurement or identification is useful in patient diagnosis or clinical management. Tumor markers are produced either by the tumor itself or by the body in response to the presence of cancer. But most of the tumor marker are also present at lower levels in healthy subjects and are not elevated in every person with cancer, especially in the early stages of the disease. Furthermore, tumor markers are in general specific to one or several particular types of cancer.

Examples of such tumor markers include prostate-specific antigen (benign prostate conditions or malignant growth in the prostate), prostatic acid phosphatase (prostate cancer), CA 125 (ovarian cancer cells), carcinoembryonic antigen (colorectal cancer, melanoma, lymphoma, and others, but also noncancerous conditions), alpha-fetoprotein (primary liver cancer or germ cell cancer, but also noncancerous conditions), human chorionic gonadotropin (choriocarcinoma, but also pregnancy and marijuana use), CA 19-9 (colorectal, pancreatic, stomach, and bile duct cancer), CA 15-3 (mainly breast cancer), CA 27-29 (breast cancer and other cancers but also a series of noncancerous conditions), lactate dehydrogenase (many types of cancer as well as many other diseases) and neuron-specific enolase (neuroblastoma and small cell lung cancer).

A preferred marker would be a marker that would give a positive result only in patients with cancer, that could be used for many different types of cancer, that would correlate with stage and response to treatment and that was easily and reproducibly measured. As detailed above, the known tumor markers do not meet these criteria.

Surprisingly, it has been found that piwi-like 2 (piwil2) is present in many cancer cells but absent in normal (i.e. noncancerous) cells other than testicular cells. Accordingly, piwil2 is a novel tumor marker for the diagnosis of cancer, which can be used in a broad range of application, i.e. for the diagnosis of a diversity of cancer.

In contrast to the markers already known piwil2 is not present in normal cells other than testicular cells, but only in cancer cells. Thus, the piwil2 tumor marker provides a very specific tool for identifying and diagnosing cancer, as positive results for a particular organ or tissue are obtained only in patients with cancer but not in healthy subjects. This reduces the measurement inaccuracy. With the tumor marker of the state of the art intensity levels are to be compared to decide whether a subject is suffering from cancer, because the respective tumor marker is elevated in the case of cancer, but also present, if the tested subject is healthy.

Additionally, it can be used for many different types of cancers. This fact reduces the effort required for diagnosing cancer, because only one type of test has to be carried out for diagnosing different cancers, which is time-saving and more economical than the tests presently known.

Furthermore, piwil2 is especially usable in early detection of cancer, since piwil2 plays a role in early stages of tumorigenesis. Being a not so expensive and broad diagnosis method of cancer, it may be used for treatment monitoring and post-treatment recurrence check. Additionally, Piwil2 enables the screening to find anticancer products. It may also be used as general target for cancer therapy.

Accordingly, a first aspect of the present invention is a method of diagnosing cancer other than testicular cancer comprising the step of:
(a) determining the amount of piwi-like 2 (piwil2) in a subject-derived sample,
wherein an increased amount of piwil2 in the sample in comparison with a control value is indicative for cancer.

The piwil2 gene, which is also known as HILI, mili, PIWIL1L or FLJ10351, is a member of the piwi gene family. The piwi genes represent a class of genes known to be required for stem cell self-renewal in diverse organisms (Cox et al., Development 2000; 127:503-14). They are defined by a conserved architecture with the PAZ motif in the middle and piwi motif in the C-terminal region and play essential roles in stem cell self-renewal, gametogenesis, and RNA interference in diverse organisms ranging from Arabidopsis to human.

In the Drosophila ovary, piwi is required in somatic signaling cells to maintain germline stem cells (Cox et al., supra). Piwi has a role as a cell-autonomous promoter of germline stem cell division. Removing piwi protein from single germline stem cells significantly decreases the rate of their division (Cox et al., supra). Overexpression of piwi in somatic cells causes an increase both in the number of germline stem cells and the rate of their division. Thus, in Drosophila, piwi is a key regulator of stem cell division - its somatic expression modulates the number of germline stem cells and the rate of their division, while its germline expression also contributes to promoting stem cell division in a cell-autonomous manner (Cox et al., supra).

In mammals, piwi genes are expressed specifically in testis and play a key role in spermatogenesis (Kuramochi-Miyagawa et al., Mech Dev. 2001; 108:121-33). In the mouse genome, two piwi homologs have been identified (Miwi and Mili: Miwi like or Piwil2). Miwi-null mice do not complete spermatogenesis, but arrest occurs at the beginning of the round spermatid stage, significantly downstream of the germline stem cell division stage (Deng and Lin H, Dev Cell. 2002; 2:819-30). The Mili-null mice showed arrest of spermatogenesis at the spermatocyte stage, which is reminiscent of the phenotype of Mvh (mouse vasa homolog)-null mice (Kuramochi-Miyagawa, Development 2004; 131:839-49). In human, four members of this family have been identified: Piwill (hiwi), Piwil2 (hili), Piwil3 and Piwil4 (hiwi2) (Sasaki et al., Genomics 2003; 82:323-30). Human Piwill (hiwi) is specifically expressed in both normal and malignant spermatogenic cells in a maturation stage-dependent pattern, in which it might function in germ cell proliferation (Qiao et al., Oncogene 2002; 21:3988-99). It was also demonstrated that Piwill (hiwi) is expressed in a variety of primitive hematopoietic cells and may play a role in determining or regulating hematopoietic stem cell development (Sharma et al., Blood 2001;97:426-34).

The human piwil2 gene maps on chromosome 8, at 8p21.3 and it covers 80.88 kb, from 22188772 to 22269647 (NCBI build 35, hg17, August 2004), on the direct strand. The complete mRNA and protein sequences are accessible at the National Center for Biotechnology Information (Accession No: AB079367; human mRNA BG025995; mouse mRNA AF285585). Other descriptions are accessible from GeneCard (PIWIL2; GC08P022154), UCSC Genome Bioinformatics (chr8: 22,168,553 - 22,289,866) and NCBI LocusLink (LocusID: 55124).

As used in the context of the present invention, the term "piwil2" refers to a nucleic acid encoding a piwil2 protein, a piwil2 protein and an anti-piwil2 antibody as defined below.

A piwil2 protein according to the present invention is any naturally occurring piwil2 protein. This includes the human piwil2 protein and homologues thereof of different species. Examples of piwil2 proteins of non-human species include without limitation piwil2 homologues of *Pan troglodytes* (LOC472711), *Mus musculus* (Piwil2), *Rattus norvegicus* (LOC306011), *Anopheles gambiae* (1279829) and *Caenorhabditis elegans* (4H292). Accordingly, the present invention can be carried out using samples of different animals and by determining the amount of the respective piwil2 protein. In a preferred embodiment, the subject is a mammal, more preferably a human.

A nucleic acid encoding piwil2 refers to a nucleic acid, e.g. an mRNA, cDNA or DNA, that encodes a piwil2 protein defined as detailed above. An anti-piwil2 antibody refers to an antibody to a piwil2 protein as defined herein. Furthermore, the term anti-piwil2 antibody encompasses an antigenically reactive fragment thereof, the fragment still being capable of binding specifically to the piwil2 protein. In accordance with the present invention, a fragment of the antibody (e.g., Fab, Fc, etc.) can be obtained from the antibodies produced as described herein, by methods which include digestion with enzymes, such as pepsin or papain, and/or cleavage of disulfide bonds by chemical reduction. Binding of the fragment to piwil-2 can be compared with the binding of the anti-piwil2 antibody. The fragment is an antigenically reactive fragment, if the binding of the fragment mounts to at least 30 %, more preferably at least 50 %, still more preferably at least 80 % and most preferably at least 90 % of that of a complete anti-piwil2 antibody.

The nucleic acid, the protein and the antibody can be fragments of the natural occurring molecules. The fragments can be either degradation products of catabolic pathways in the organism, from which the sample is derived, or degradation products resulting from the methods used for e.g. pre-treatment or measurement. However, the amount of the degradation product reflects the amount of piwil2 in the subject or organ the sample has been taken from.

In one embodiment, the amount of piwil2 in the sample in comparison with the control value refers either to the presence (indicative for cancer) or absence (control value and healthy subject) of piwil2. In another embodiment, the amount of piwi12 in the sample in comparison with the control value refers to either an increased value (indicative for cancer) or a basal value (control value and healthy subject).

Piwil2 in the sample can be determined using different techniques depending on the nature of the sample. Assay techniques that can be used to determine the amount of piwil2 in a sample derived from a subject are well-known to those of skill in the art. Such assay methods include, without limitation, nucleic acid amplification method, hybridization method, enzyme immunoassay, fluorescence immunoassay, luminescent immunoassay or protein bioassay which include membrane, solution, or chip-based technologies for the detection and/or quantification of nucleic acid or protein, radioimmunoassays, reverse transcriptase PCR (RT-PCR) assays, immunohistochemistry assays, in situ hybridization assays, competitive-binding assays, Western Blot analyses, ELISA assays and proteomic approaches, two-dimensional gel electrophoresis (2D electrophoresis) and non-gel based approaches such as mass spectrometry or protein interaction profiling. Among these, ELISAs are frequently preferred to diagnose a protein in biological fluids.

In particular, quantification of RNA level can be performed using quantitative real-time PCR and on protein level can be performed using a defined concentration of in vitro generated and purified Piwil2 protein.

In one embodiment of the invention piwil2 is the mRNA encoding a piwil2 protein as defined herein. Methods of determining the amount of mRNA are known to the person skilled in the art and exemplarily listed above. In a more preferred embodiment the amount of mRNA encoding a piwil2 protein is determined by a hybridization method or a nucleic acid amplification method. Such methods are known to the artisan and include the dot blot hybridization method, the Northern hybridization method, the RT-PCR method and other nucleic acid methods, such as ligase chain reaction (LCR) and nucleic acid sequence based amplification (NASBA). For example, Northern hybridization analysis uses probes which specifically recognize mRNA encoding the piwil2 protein. A solution hybridization method for the quantification of specific mRNAs is described e.g. in Tenhunen et al. (Genet Anal Tech Appl. 1990; 7:228-233.). This assay utilizes complementary RNA probes prepared by in vitro transcription, sandwich hybridization in solution, and affinity-based hybrid collection. It can be used for crude biological samples without purifying mRNAs. Alternatively, mRNA is measured using reverse-transcription-based PCR assays. In RT-PCR, the mRNA is first reverse transcribed to complementary DNA (cDNA) with use of the enzyme reverse transcriptase and a primer specific for piwi12; the cDNA is then amplified as in a standard PCR reaction. RT-PCR can thus reveal by amplification the presence of a single species of mRNA, i.e. mRNA encoding a piwil2 protein.

Examples of methods of determining the amount of mRNA encoding a piwil2 protein are described in Examples 1.3 and 1.4.

Various haematological malignancies display characteristic chromosomal rearrangements. Often these translocations (transfer of a part of one chromosome to a non-homologous chromosome) target transcription factor or tyrosine kinase genes and result in dysregulated expression or production of a fusion protein with oncogenic properties. Activation of Piwil2 in cancer cells e.g. can be a result of genetic rearrangement so that Piwil2 is activated by promoter or enhancer of other gene or Piwil2 in cancer cells is a recombinant fusion protein. This can be tested by sequencing of entire Piwil2 mRNA in various cancer types. Thereafter, a RT-PCR or PCR based on primer located in Piwil2 gene and fused mRNA can be used as a diagnostic tool.

A preferred primer is a piwil2-derived primer designed according to and characterized by the following criteria:

It matches 100% with the nucleic acid to be amplified, e.g. the mRNA encoding the piwil2 protein. The size of the primer is 18-30 nucleotides long, preferably 20-25 nucleotides long. The 3' end base is preferably G or C. The GC content amounts to 40-60%. Self-complementarity should be avoided to minimize primer secondary structure and primer dimer formation. The melting temperature is 50 to 65°C.

More preferably, the primer is located in the coding region which has no homology with other piwi genes such as the genes coding for Piwill (hiwi), Piwil3 and Piwil4 (hiwi2).

Even more preferably, the primer contains or consists of the sequences of SEQ ID NO:1, SEQ ID NO:2, SEQ ID NO:3 and SEQ ID NO:4 or homologues thereof.

In another embodiment piwil2 is the piwil2 protein as defined above. Methods of determining the amount of a protein are well known to the person skilled in the art and detailed above. In a preferred embodiment the amount of piwil2 protein is determined by using binding proteins capable of binding specifically to the piwil2 protein. In a more preferred embodiment the binding protein is an antibody or antibody fragment, especially an anti-piwil2 antibody recognizing an epitope of the piwil2 protein showing no homology to other piwi proteins such as Piwill (hiwi), Piwil3 and Piwil4 (hiwi2), particularly wherein the epitope is located N-terminally, more particularly wherein the antibody recognizes epitope "DPVRPLFRG PTPVHP" (SEQ ID NO:5) of the piwil2 protein or a homologue thereof. Such an antibody can be prepared as known by a person skilled in the art or as exemplified in the Examples (see Example 1.8). N-terminally refers to that portion of the protein which is located at the N-terminus and preferably encompasses 1/3 of the total protein, more preferably 1/4, still more preferably 1/5, even more preferably 1/6 and most preferably 1/10 of the total protein. For generation of an antibody a stretch of 10-15 amino acid is in general sufficient to induce an immunoreaction and results in generation of an antibody.

As used herein, the term "homology" refers to concordance of an amino acid sequence with another amino acid sequence or of a polynucleotide sequence with another polynucleotide sequence of at least 70% or preferably, at least 80%, when such sequences are arranged in a best fit alignment. In the case of nucleotide sequences, the term also implies that the nucleotide sequence in question is capable of being detected in a screening assay by a hybridization probe derived from the nucleotide sequence encoding a piwi12 proteinunder moderate stringency conditions. (Ausubel, F. M., et al., in Current Protocols in Molecular Biology, John Wiley and Sons, Inc., Media Pa. (1992)).

An "alignment" refers to the arrangement of two or more amino acid or nucleic acid sequences in such a way as to align areas of the sequences sharing common properties. The degree of relatedness or homology between the sequences is predicted computationally or statistically based on weights assigned to the elements aligned between the sequences.

Percent (%) identity, with respect to two amino acid sequences, refers to the percent of residues that are identical in the two sequences when the sequences are optimally aligned. Optimal alignment is defined as the alignment giving the highest percent identity score. Such alignments can be performed using the "GENEWORKS" program. Alternatively, alignments may be performed using the local alignment program LALIGN with a ktup of 1, default parameters and the default PAM. In the context of the present invention, when it is stated that a protein or nucleic acid has 80% identity to a given sequence, it is implicit that this refers to the entire sequence of the longer of the two proteins.

The methods for determining the amount of piwil2 protein include ELISA, RIA, western blot or quantitative analytical techniques such as spectroscopy or flame chromatography. The antibody necessary for an antibody-based method can be prepared according to procedures known to the one of skill in the art. Antibodies which can be used include polyclonal, monoclonal and omniclonal antibodies and antibodies prepared via molecular biology techniques.

Antibody-based assay initially comprise preparing an antibody, if not readily available from a commercial source, specific to the piwil2 protein, preferably a monoclonal antibody. In addition a reporter antibody generally is prepared which binds specifically to the primary antibody. The reporter antibody is attached to a detectable reagent such as radioactive (RIA), fluorescent or enzymatic reagent (ELISA), for example horseradish peroxidase enzyme or alkaline phosphatase. The intensity of the signal of the detectable marker (e.g. absorbance or intensity of color) reflects the amount of piwil2 protein in the sample.

Examples of methods of determining the amount of a piwil2 protein are described in Example 1.8.

A competition assay may be employed wherein antibodies specific to the piwil2 protein attached to a solid support and labeled piwi12 and a sample derived from the subject to be tested are passed over the solid support and the amount of label detected attached to the solid support can be correlated to a quantity of piwil2 protein in the sample.

In another embodiment piwil2 is an anti-piwil2 antibody as defined above. Methods of determining the amount of an antibody, e.g. by using an antibody to the anti-piwil2 antibody, are well known to the person skilled in the art. The skilled person in the art will recognize that methods listed for piwil2 proteins can be modified and employed for the determination of the amount of an anti-piwil2 antibody.

In a preferred embodiment of the invention the amount of piwi12 is determined by measuring the amount of mRNA encoding the piwil2 protein, the amount of piwil2 protein and/or the amount of anti-piwil2 antibody in the sample.

The test sample used in conjunction with the invention can be any of those typically used in the art. The test sample can be taken from any source in which piwil2 can be present in case of cancer. Examples of subject-derived samples are blood, faeces, saliva, urine, a cell lysate, a tissue sample, a biopsy, a tissue lysate, a cell culture, liquor and cerebrospinal fluid. By blood it is meant to include whole blood, plasma, serum or any derivative of blood.

The test sample can be a tissue. Typically, the tissue is a tissue supposed to be cancerous and has been obtained by means of a biopsy or autopsy. Such tissue can include bone marrow, lymph nodes, skin, and any organ that may develop cancerous cells e.g. tongue or mouth; pharynx; the digestive system such as esophagus, stomach, small intestine, colon, rectum, anus, anal canal, anorectum, liver, intrahepatic bile duct, gallbladder or pancreas; the respiratory system such as larynx, lung or bronchus; bones and joints; a soft tissue including heart; the skin such as melanoma-skin or non-epithelial skin; the breast; the genital system such as uterine cervix, uterine corpus, ovary, vulva, vagina, prostate or penis; urinary system such as urinary bladder, kidney, renal pelvis or ureter; an eye; the orbit; the brain; the nervous system; endocrine system such as thyroid; or any other site. Examples of tissues are listed in Examples 1.4 and 1.8.

If necessary or desired, the subject-derived sample can be pre-treated, e.g. purified or separated, before the amount of piwil2 in the sample is determined. Such methods are known to the one of skill in the art and include homogenization, centrifugation, differential centrifugation, filtration, ultrafiltration, dialysis, precipitation, chromatographic separation, such as affinity chromatography or ion exchange chromatography, and electrophoresis such as gel electrophoresis.

In a preferred embodiment the subject-derived sample to be tested in the method of the present invention is a blood sample, tissue sample, urine sample, saliva sample, a serum sample or a plasma sample.

The control value can be obtained by determining the amount of piwi12 in sample derived from a healthy subject. Healthy subject in this context refers to a subject not suffering from the cancer to be diagnosed (e.g. when using a tissue sample) or not suffering from cancer at all (e.g. when using a urine sample or blood sample). The control value can be determined either together e.g. simultaneously with the subject-derived sample to be tested or at a different time.

In a preferred embodiment piwil2 is not present in the sample of a healthy subject as in general Piwil2 is not detectable in normal tissues other than testis. Detection of Piwil2 is therefore in general an evidence for malignant transformation of tissues. The higher expression of Piwil2 the more malignant this tissue is. In this case e.g. the background signal of the measuring method can be used as control value. In view of the present disclosure, the skilled artisan can readily determine an appropriate control value.

Within the context of the present invention, the amount of piwil2 in the sample is increased in comparison with a control, if the amount of piwil2 in the sample is significantly higher than the control value. The person skilled in the art knows statistical procedures to assess whether two values are significantly different from each other such as Student's t-test or chi-squared test. In a preferred embodiment the amount of piwil2 in the sample amounts to at least 110 %, preferably to at least 125 %, more preferably to at least 150 %, 160 %, 170 %, 180 % or 190 %, still more preferably to at least 200 % and most preferably to least 300 % of the control value.

Cancer cells are, by definition, abnormal and contain proteins which should be recognized by the immune system as foreign since they are not present in normal tissues. However, the immune system often seems to ignore this abnormality and fails to attack tumors. Examples of cancer are aids-related cancer, Karposi's sarcoma, brain tumor, cerebellar astrocytoma, cerebral astrocytoma/malignant glioma, ependymoma, medulloblastoma, supratentorial primitive neuroectodermal tumors and pineoblastoma, visual pathway and hypothalamic glioma, breast cancer, anal cancer, extrahepatic bile duct cancer, gastrointestinal carcinoid tumor, colon cancer, esophageal cancer, gallbladder cancer, liver cancer, pancreatic cancer, rectal cancer, small intestine cancer, stomach (gastric) cancer; adrenocortical carcinoma, gastrointestinal carcinoid tumor, islet cell carcinoma (endocrine pancreas), parathyroid cancer, pheochromocytoma, pituitary tumor, thyroid cancer, intraocular melanoma, retinoblastoma, bladder cancer, kidney (renal cell) cancer, penile cancer, prostate cancer, renal pelvis and ureter cancer, urethral cancer, Wilm's tumor, cervical cancer, endometrial cancer, gestational trophoblastic tumor, ovarian epithelial cancer, ovarian low malignant potential tumor, uterine sarcoma, vaginal cancer, vulvar cancer, hypopharyngeal cancer, laryngeal cancer, lip and oral cavity cancer, metastatic squamous neck cancer with occult primary, nasopharyngeal cancer, oropharyngeal cancer, paranasal sinus and nasal cavity cancer, parathyroid cancer, salivary gland cancer, acute lymphoblastic leukemia, acute lymphoblastic leukemia, acute myeloid leukemia, acute myeloid leukemia, chronic lymphocytic leukemia, chronic myelogenous leukemia, hairy cell leukemia lymphoma, AIDS-related lymphoma, cutaneous T-cell lymphoma, Hodgkin's lymphoma, mycosis fungoides, non-Hodgkin's lymphoma, primary central nervous system lymphoma, Sezary syndrome, cutaneous T-cell lymphoma, Waldenström's macro-globulinemia, chronic myeloproliferative disorders, multiple myeloma/plasma cell neoplasm, myelodysplastic syndromes, myelodysplastic/myeloproliferative disease, non-small cell lung cancer, small cell lung cancer, ewing's tumor, osteosarcoma/malignant fibrous histiocytoma of bone, rhabdomyosarcoma, soft tissue sarcoma, uterine sarcoma, neuroblastoma, pituitary tumor, primary central nervous system lymphoma, malignant mesothelioma, thymoma and thymic carcinoma, melanoma, Merkel cell carcinoma and skin cancer. The terms cancer and tumor are used interchangeable.

In a preferred embodiment the method of the invention is used in early detection, to monitor or assess a cancer's response to treatment and/or to check for recurrence.

As used herein "early detection" refers to a medicinal examination for the prevention and early diagnosis of cancer and means the detection of cancer in early stages of cancer formation and tumorigenesis. In general the examination is carried out before the subject examined displays symptoms typical for the prevailing cancer. The method can be used for screening a healthy population or a high risk population for the presence of cancer, i.e. in order to identify the individuals of the healthy having cancer optionally without having symptoms. Cancer formation is a multistage process involving tumor initiation, promotion, conversion, and progression. Most current methods of cancer early detection, such as mammography or cervical cytology, are based on anatomic changes in tissues or morphologic changes in cells. The advantage of the use of piwil2 for early cancer detection is that cancer formation can be detected at earlier stages and therefore the chance for the uses of preventing procedures will be increased. For example prostate cancer five-year survival is nearly 100% when the disease is diagnosed at a local or regional stage, but poor when diagnosed with distant metastases (32.6%). The detection of Piwil2 in most cancers and its position in hierarchy of gene activation (Stat3Bc1-x, Stat3/CyclinD1 pathway) make piwil2 highly suitable for early detection of many cancers.

Furthermore, diagnosis or diagnosing according to the present invention also includes imaging of cancer. For imaging e.g. antibodies can be used as well. For example, antibody-chelators labeled with Indium-111 have been described for use in the radioimmunoscintographic imaging of carcinoembryonic antigen expressing tumors (Sumerdon et. al. Nucl. Med. Biol. 1990 17:247-254). In particular, these antibody-chelators have been used in detecting tumors in patients suspected of having recurrent colorectal cancer (Griffin et al. J. Clin. One. 1991 9:631-640). Antibodies with paramagnetic ions as labels for use in magnetic resonance imaging have also been described (Lauffer, R. B. Magnetic Resonance in Medicine 1991 22:339-342). Antibodies directed against piwil2, in particular piwil2 protein, can be used in a similar manner. Labelled antibodies which specifically bind piwil2 can be injected into patients suspected of having cancer for the purpose of diagnosing or staging of the disease status of the patient. The label used will be selected in accordance with the imaging modality to be used. For example, radioactive labels such as Indium-111, Technetium-99m or Iodine-131 can be used for planar scans or single photon emission computed tomography (SPECT). Positron emitting labels such as Fluorine-19 can be used in positron emission tomography. Paramagnetic ions such as Gadolinium (III) or Manganese (II) can be used in magnetic resonance imaging (MRI). Presence of the label, as compared to imaging of normal tissue, permits determination of the spread of the cancer. The amount of label within an organ or tissue also allows determination of the presence or absence of cancer in that organ or tissue.

The method can also be used to monitor or assess a cancer's response to a treatment by monitoring the course in a patient in remission or while being treated, e.g. while receiving surgery, radiation, or chemotherapy. To monitor or assess cancer's response to treatment the amount of piwil2 is determined concurrently with and/or before and after the treatment of a cancer. The treatment can be any treatment such as chemotherapy, radiation, surgery, immunotherapy or a combination thereof. The amount of piwi12 is determined at least twice or preferably repeated at regular intervals such as every week, every month, every 2, 3, 4 6 or 12 months in order to detect a changed amount of piwil2. A reduction of piwil2 in the sample taken at a later date in comparison with the sample taken at a earlier date is indicative for a subject's response of the therapy or treatment chosen. If the amount of piwil2 rises, it may indicate that the cancer is growing.

Furthermore, the method can also be used to check for recurrence of a cancer. A subject that is deemed to be cured from cancer can be examined and/or controlled using the method of the invention. Therefore, a sample of the subject is taken at least once after (successful) therapy or from time to time and the amount of piwil2 in the subject's sample is determined. No decrease of the amount of piwil2 compared to previous values (if the cancer has still been present at the time, the sample was taken) or an increased amount of piwi12 in comparison with the control value is indicative for the recurrence of a cancer.

Another aspect of the present invention is the use of an indicator of piwil2 for the diagnosis of cancer other than testicular cancer.

Indicators detecting piwi12 can be used e.g. in laboratory tests as part of a routine checkup to identify possible changes in a person's health before any symptoms appear. Additionally, laboratory tests play an important role in diagnosis when a person has symptoms. In addition, tests may be used to help plan a patient's treatment, evaluate the response to treatment, or monitor the course of the disease over time (see above).

A laboratory test is a medical procedure in which a sample as described above is checked for certain features, in the present case for the presence and amount of piwil2. The indicators of piwil2 can be used in laboratory test samples to determine whether the amount of piwil2 falls within normal ranges, i.e. increased or not increased in comparison with the control value. Samples also may be checked for changes from previous tests. Control values might be given as a specific number or preferably as a range, because normal values vary from person to person. What is normal for one person may not be normal for another person. Many factors (including the patient's sex, age, race, medical history, and general health) can affect test results. Sometimes, test results are affected by specific foods, drugs the patient is taking, and how closely the patient follows pre-test instructions.

The information obtained from laboratory tests using a piwil2 indicator can help physicians decide whether other tests or procedures are needed to make a diagnosis. The information can also help the physician develop or revise a patient's treatment plan.

Piwi12 is absent in normal cells other than testicular cells as understood by the artisan, but present in cancerous cells. Accordingly, an indicator of piwil2 that indicates the presence of piwil2 in a sample can be used for the diagnosis of cancer other than testicular cancer. In a preferred embodiment of the invention piwi12 is only present in the sample in case of cancer but not in a healthy subject. Accordingly, the presence of piwil2 in the sample is indicative for cancer.

As indicator any of the systems or compounds for detecting or measuring piwil2 mentioned above can be used.

If the indicator is a compound, the indicator compound or a compound interacting with the indicator needs to be detected. In one embodiment the indicator compound or the compound interacting with the indicator can be linked to a label or marker. Techniques for linking the compound to a marker or label are known by those skilled in the art and can be used in various nucleic acid and amino acid assays. Suitable marker or labels which can be used for ease of detection include radionuclides, enzymes, and fluorescent, chemiluminescent, or chromogenic agents, as well as substrates, cofactors, inhibitors, magnetic particles, and the like. These can be coupled e.g. to a protein binding to piwil2 or an antibody thereto. Means for producing labeled hybridization or PCR probes for detecting sequences related to piwi12 include oligolabeling, nick translation, end-labeling, or PCR amplification using a labeled nucleotide.

In a preferred embodiment of the invention indicator is a nucleic acid, in particular a primer pair and/or a probe, particularly comprising a piwil2-based primer, especially a primer as defined SEQ ID NO:1, SEQ ID NO:2, SEQ ID NO:3 or SEQ ID NO:4 or a homologue thereof. The probe or primer can be labeled during the procedure used for the detection e.g. during PCR by using e.g. labeled nucleotides. Nucleotides can be labeled e.g. with ³²P, ³H or ¹⁴C. Methods of designing and producing probes and/or primers e.g. by using suitable design software are known to the one of skill in the art. Examples of such methods, in particular PCR, are described in Example 1.4.

In another preferred embodiment of the invention the indicator is an antibody or antibody fragment, especially an anti-piwil2 antibody recognizing an epitope of the piwil2 protein showing no homology to other piwi proteins, particularly wherein the epitope is located N-terminally, more particularly wherein the antibody recognizes epitope "DPVRPLFRG PTPVHP" of the piwi12 protein or a homologue thereof. Such antibodies can be polyclonal, monoclonal, or omniclonal or prepared by molecular biology techniques. Antibodies can be labeled with a variety of detectable labels including, but not limited to, radioisotopes, enzymes and paramagnetic metals. An examples of such an antibody and its production is described in Example 1.8.

In another preferred embodiment of the use according to the invention the piwil2 to be detected is an mRNA encoding the piwil2 protein, a piwi12 protein and/or an anti-piwi12 antibody, wherein these terms are as defined above. Determination is carried out as detailed above.

Another aspect of the present invention is the provision of a kit suitable for diagnosing a cancer other than testicular cancer, comprising
(i) a primer pair and/or a probe suitable for determining the amount of mRNA encoding piwi12, in particular comprising a piwil2-based primer, especially a primer as defined in SEQ ID NO: 1, SEQ ID NO:2, SEQ ID NO:3 or SEQ ID NO:4 or a homologue thereof; and/or
(ii) an antibody or antibody fragment, especially an anti-piwil2 antibody recognizing an epitope of the piwil2 protein showing no homology to other piwi proteins, particularly wherein the epitope is located N-terminally, more particularly wherein the antibody recognizes epitope "DPVRPLFRG PTPVHP" of the piwil2 protein or a homologue thereof.

The kit encompasses a primer pair, a probe and/or antibodies which are defined as above. The primer and/or the probe are intended to specifically identify one or more nucleic acids encoding piwil2 proteins by having homologous nucleic acid sequences complementary to a portion of the DNA or preferably RNA encoding piwi12. Antibodies are intended to identify proteins, i.e. the piwil2 protein or an anti-piwil2 antibody. Specifically binding in the context of the present invention refers e.g. to an antibody binding to the piwil2 protein or the anti-piwi12 antibody having an association constant K of at least 10⁷ (mol/l)⁻¹, preferably having an association constant K in the range of from about 10⁸ (mol/l)⁻¹ to about 10¹² (mol/l)⁻¹. The association constant K can be determined using e.g. equilibrium dialysis and Scatchard plots.

In one embodiment of the invention the primer or antibody may be either already bound to a solid matrix or packaged separately with reagents for binding them to the matrix. Additionally the kit may comprise suitable buffers, control formulations, and/or a detectable label. Instructions (e. g. written, tape, VCR, CD-ROM, etc.) for carrying out the assay may be included in the kit. The assay may, for example, be in the form of a Northern hybridization or an ELISA as known in the art. Alternatively, the kit may contain a nucleic acid substrate array comprising one or more nucleic acid sequences. The nucleic acids on the array specifically identify piwil2 and optionally other tumor markers. The presence of piwil2 and other tumor markers is identified by virtue if binding to the array. The substrate array can be on, e. g., a solid substrate, e. g., a "chip" as described in U. S. Patent No. 5,744,305.

The method of diagnosing of the invention, the kit of the invention and the use of the invention can be used for any purpose. This includes e.g. uses in laboratory scale, for research reasons or in an automatic analyzer.

Another aspect of the present invention refers to a method of screening for an inhibitor of piwil2 for use in the treatment or prevention of cancer comprising the steps of:
(a) exposing a test cell expressing the piwil2 protein to a test compound; and
(b) comparing the rate of apoptosis or the rate of proliferation of the test cell with that of a control cell,
   wherein an increased rate of apoptosis or an decreased rate of proliferation of the test cell in comparison with that of the control cell is indicative for an inhibitor of piwi12.

An inhibitor of piwil2 is a compound inhibiting the action of piwil2, in particular the piwil2 protein. The compound may be any molecule including a protein or fragment thereof, a small molecule, or even a nucleic acid molecule. These can be natural compounds, synthetic compounds or synthetic modifications of natural compounds. The compounds may include fragments or parts of naturally-occurring compounds, or may be found as active combinations of known compounds, which are otherwise inactive. It is proposed that compounds isolated from natural sources, such as animals, bacteria, fungi, plant sources, including leaves, flowers and bark, and marine samples may be assayed as candidates for the presence of potentially useful pharmaceutical agents. It will be understood that the pharmaceutical agents to be screened could also be derived or synthesized from chemical compositions or man-made compounds as well as biotechnologically produced. Thus, it is understood that the candidate substance identified by the present invention may be peptide, polypeptide, polynucleotide, an antibody, e.g. an anti-piwil2 antibody, a nucleic acid such as e.g. an mRNA, an oligonucleotide, an siRNA an antisenseRNA, a microbody as defined below or a small organic compound (< 2000 molecular weight, preferably < 1000 molecular weight, more preferably < 600 molecular weight).

The piwi12 protein stimulates cell proliferation and prevents apoptosis. Accordingly, an inhibitor of piwi12 interacts with piwil2 thereby increasing the rate of apoptosis and decreasing the rate of proliferation in a cell or cell population. The inhibitor can be identified due to its influence on proliferation and/or apoptosis. Accordingly, a test cell is exposed to a test compound and the rate of apoptosis and/or the rate proliferation of the test cell is compared with that of a control cell. The test compound can be incorporated into the cell by receptors, shuttles, channels, endocytosis or through the cell membrane due to its lipophilicity or membrane pores due to its size. The identified inhibitors of piwil2 are useful compounds for the therapy and prophylaxis of cancer.

In one embodiment of the invention the test cell is a cell expressing piwil2, whereas the control cell is a cell not expressing piwil2. Both cells are preferably of the same type, i.e. the same origin, but differently genetically modified, e.g. transfected with e.g. different vectors, i.e. with and without piwil2 gene. Any cell, in particular any animal cell, preferably a mammalian cell or a human cell can be used. Examples of such cells and their production are described in Examples 1.1 and 1.2. Both cells can be incubated with the same test substance and proliferation and/or apoptosis are compared. An increased rate of apoptosis or a decreased rate of proliferation of the test cell (cell expressing piwil2) in comparison with that of the control cell (cell not expressing piwil2) are indicative for an inhibitor of piwil2.

In another embodiment the test cell and the control cell are genetically identical, but only test cell is incubated with the test substance. Thereafter apoptosis and/or proliferation of both test and control cells are compared. An increased rate of apoptosis or a decreased rate of proliferation of the test cell (cell expressing piwi12 and incubated with the test compound) in comparison with that of the control cell (cell expressing piwil2 but no incubation with the test compound) is indicative for an inhibitor of piwil2.

Examples of apoptosis assays and proliferation assays are described in Examples 1.5 and 1.6, which are to be modified in that the cells are to be incubated with the test compound. The skilled person knows how to incubate cells in a suitable manner by choosing e.g. an appropriate time, temperature, concentration, solvent etc.

In a preferred embodiment are populations of test cells and control cells are compared to achieve results of appropriate statistical evaluation. A cell population refers to a group of genetically identical cells. More preferably each cell population encompasses at least 1,000 cells, even more preferably more than 2,000 cells and most preferably more than 5,000 cells.

The rate of apoptosis and proliferation e.g. can be determined by cell counts using an appropriate counting cell chamber, a photometer for determination of density of a cell suspension or automatic cell counters.

In a preferred embodiment of the invention the test compound is provided in the form of a chemical compound library. Chemical compound libraries include a plurality of chemical compounds and have been assembled from any of multiple sources, including chemically synthesized molecules and natural products, biotechnological products or have been generated by combinatorial chemistry techniques. They are especially suitable for high throughput screening. They may be comprised of chemical compounds of a particular structure or compounds of a particular creature such as a plant, an animal or an microorganism. One may simply acquire, from various commercial or non-commercial sources, e.g. small molecule libraries that are believed to meet the basic criteria for useful drugs. Screening of such libraries, including combinatorially generated libraries, is a rapid and efficient way to screen large number of related (and unrelated) compounds for activity. In the context with the present invention the chemical compound library is preferably a library comprising proteins, polypeptides, microproteins (Selecore, Göttingen, Germany) or small organic compounds (< 2000 molecular weight, preferably < 1000 molecular weight, more preferably < 600 molecular weight).

Microproteins are proteins defined by the following characteristics: Small size, typically less than 50 amino acids, defined structure based on the intra-molecular disulfide bonds, highly stable and resistant to heat, pH, and proteolytic degradation and pharmacological activity. Large randomized libraries (CoLibry) and selection procedures (MoDis, HT-Select) to identify and validate (RanCom) high-affinity binders against a chosen target molecule as well as established technologies to directly generate microproteins or Microbodies™ against selected targets are available from Selecore (Göttingen, Germany).

In another preferred embodiment of the screening method of the invention the method is arranged in form of a high-through put screening. In such a system advantageously the screening method is automated and miniaturized; in particular it uses miniaturized wells and microfluidics controlled by a robot.

In still another preferred embodiment of the screening method of the invention the method further comprises the step of incorporating the inhibitor of piwil2 into a therapeutic or prophylactic composition. The composition is used for the therapy or prophylaxis of cancer. For the medication the inhibitor of piwil2 or its pharmaceutically acceptable salt should to be in a pharmaceutical dosage form in general consisting of a mixture of ingredients such as pharmaceutically acceptable carriers or auxiliary substances combined to provide desirable characteristics.

The formulation comprises at least one suitable pharmaceutically acceptable carrier or auxiliary substance. Examples of such substances are demineralised water, isotonic saline, Ringer's solution, buffers, organic or inorganic acids and bases as well as their salts, sodium chloride, sodium hydrogencarbonate, sodium citrate or dicalcium phosphate, glycols, such a propylene glycol, esters such as ethyl oleate and ethyl laurate, sugars such as glucose, sucrose and lactose, starches such as corn starch and potato starch, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils such as groundnut oil, cottonseed oil, corn oil, soybean oil, caster oil, synthetic fatty acid esters such as ethyl oleate, isopropyl myristate, polymeric adjuvans such as gelatin, dextran, cellulose and its derivatives, albumins, organic solvents, complexing agents such as citrates and urea, stabilizers, such as protease or nuclease inhibitors, preferably aprotinin, ε-aminocaproic acid or pepstatin A, preservatives such as benzyl alcohol, methyl- and/or propylparabenes, oxidation inhibitors such as sodium sulphite, waxes and stabilizers such as EDTA. Colouring agents, releasing agents, coating agents, sweetening, flavouring and perfuming agents, preservatives and antioxidants can also be present in the composition. The physiological buffer solution preferably has a pH of approx. 6.0-8.0, especially a pH of approx. 6.8-7.8, in particular a pH of approx. 7.4, and/or an osmolarity of approx. 200-400 milliosmol/liter, preferably of approx. 290-310 milliosmol/liter. The pH of the medicament is in general adjusted using a suitable organic or inorganic buffer, such as, for example, preferably using a phosphate buffer, tris buffer (tris(hydroxyl-methyl)ami-nomethane), HEPES buffer ([4-(2-hydroxyethyl)piperazino]ethanesulphonic acid) or MOPS buffer (3-morpholino-1-propanesulphonic acid). The choice of the respective buffer in general depends on the desired buffer molarity. Phosphate buffer is suitable, for example, for injection and infusion solutions. Methods for formulating a medicament as well as suitable pharmaceutically acceptable carriers or auxiliary substances are well known to the one of skill in the art. Pharmaceutically acceptable carriers and auxiliary substances are a. o. chosen according to the prevailing dosage form and identified compound.

The composition can be manufactured for oral, nasal, rectal, parenteral, vaginal, topic or vaginal administration. Parental administration includes subcutaneous, intracutaneous, intramuscular, intravenous or intraperitoneal administration.

The composition can be formulated as various dosage forms including solid dosage forms for oral administration such as capsules, tablets, pills, powders and granules, liquid dosage forms for oral administration such as pharmaceutically acceptable emulsions, microemulsions, solutions, suspensions, syrups and elixirs, injectable preparations, for example, sterile injectable aqueous or oleaginous suspensions, compositions for rectal or vaginal administration, preferably suppositories, and dosage forms for topical or transdermal administration such as ointments, pastes, creams, lotions, gels, powders, solutions, sprays, inhalants or patches.

The specific therapeutically effective dose level for any particular patient will depend upon a variety of factors including the activity of the identified compound, the dosage form, the age, body weight and sex of the patient, the duration of the treatment and like factors well known in the medical arts.

Another aspect of the invention refers to the use of an anti-piwil2 antibody, an anti-piwi12 antibody fragment, a microprotein, an antisense polynucleotide such as antisense RNA or small interfering RNA (siRNA) of a polynucleotide encoding piwil2 for the manufacture of a medicament for treating or preventing cancer.

As detailed above inhibitors of piwil2 can be used for the treatment and prophylaxis of cancer. For example, in one embodiment, an antibody or fragment thereof or a microprotein which specifically binds to piwil2 protein can be raised and used for the manufacture of a medicament for in vivo treatment of patients suspected of developing cancer or suffering from cancer. Antibodies which specifically bind piwil2 protein can be injected into a patient for prophylactic or therapeutic purposes in an effective amount. By effective amount it is meant the amount or concentration of antibody needed to bind to the piwil2 protein to cause tumor shrinkage for surgical removal, or disappearance of the tumor. The inhibitory character of an anti-piwil2 antibody, an anti-piwil2 antibody fragment, a microprotein, an antisense polynucleotide or small interfering RNA (siRNA) of a polynucleotide encoding piwil2 can be tested using e.g. the screening test detailed above.

Using siRNA for gene silencing is a rapidly evolving tool in molecular biology. The person skilled in the art knows several methods for designing and for preparing siRNA, such as chemical synthesis, in vitro transcription, siRNA expression vectors, and PCR expression cassettes. Additionally, methods for designing and preparing siRNAs as well as siRNAs are provided by commercially suppliers (e.g. Qiagen, Hilden, Germany).

The anti-piwil2 antibody, an anti-piwil2 antibody fragment, a microprotein, an antisense polynucleotide or small interfering RNA of a polynucleotide encoding piwil2 can be formulated and used as detailed above for the piwi12 inhibitors.

In a preferred embodiment the method of the invention, the use of the invention or the kit of the invention refers to a cancer located at a oral cavity such as tongue or mouth; pharynx; the digestive system such as esophagus, stomach, small intestine, colon, rectum, anus, anal canal, anorectum, liver, intrahepatic bile duct, gallbladder or pancreas; the respiratory system such as larynx, lung or bronchus; bones and joints; a soft tissue including heart; the skin such as melanoma-skin or non-epithelial skin; the breast; the genital system such as uterine cervix, uterine corpus, ovary, vulva, vagina, prostate or penis; urinary system such as urinary bladder, kidney, renal pelvis or ureter; an eye; the orbit; the brain; the nervous system; endocrine system such as thyroid; or any other site.

In a more preferred embodiment the cancer is breast tumor, cervical cancer, leukemia, lymphoma, medullablastoma, rhabdomyosarcoma, colon cancer, ovarian cancer, endometrial cancer, renal cancer, gastrointestinal cancer, pancreas cancer, blood cancer, prostate cancer or lymph node tumor.

The following figures and examples further illustrate the invention but, of course, should not be construed as in any way limiting its scope.

### FIGURES

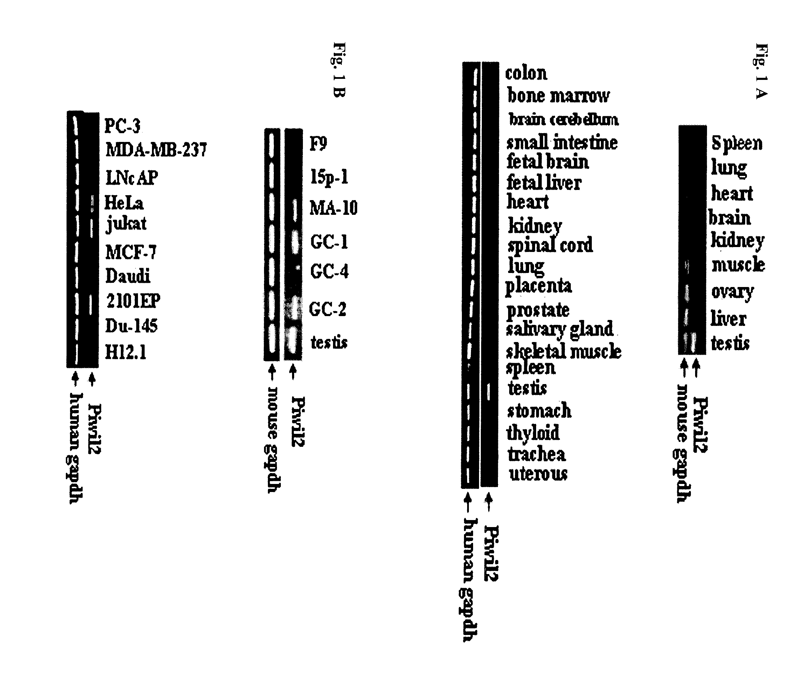

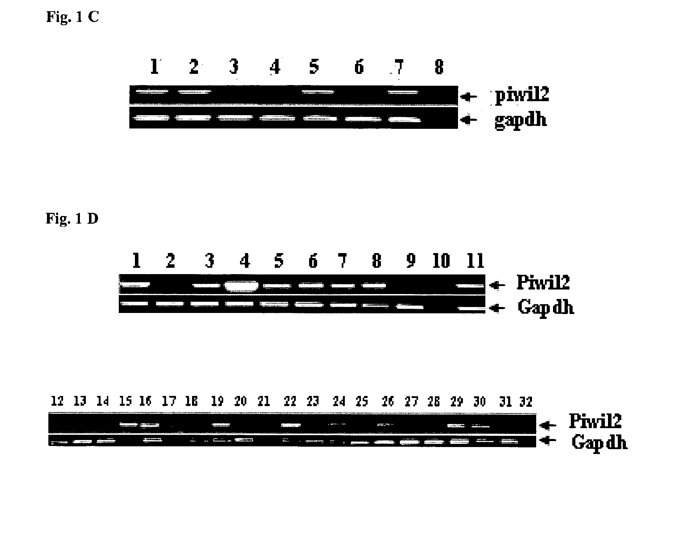

**Fig.1.** Expression analysis of Piwil2 in normal tissues, cancer cell lines and cancer tissues using RT-PCR. Mouse and human Gapdh were used as positive control. (A) Expression analysis of Piwil2 in normal mouse (top) and human (bottom) tissues, a testis-specific expression was observed. (B) Expression of Piwil2 in different cancer cell lines using RT-PCR analysis: in mouse (top), expression was obtained in transformed germ cells (GC-1 and GC-2), in transformed Leydig cell line (MA-10), and in teratocarcinoma cell line F9, but not in non-transformed spermatocyte (GC-4) and Sertoli cell (15-P1) lines. In human cancer cell lines (bottom), expression of Piwil2 was obtained in MDA-MB-237 (breast cancer), prostate cancer (PC-3, LNcAP, Du-145), HeLa (cervical cancer), Jurkat (T cell leukaemia), MCF-7 (breast adenocarcinoma), Daudi (Burkitt's lymphoma), 2101EF (embryonal carcinoma). (C) Expression of Piwil2 in different mouse cancer tissues. Three types of tumors with corresponding normal tissues were examined. Expression was detected in breast tumor (2), in medullobalstoma (5) and in rhabdomyosarcoma (7), whereas, no expression was detected in normal breast (3), cerebellum (4) and skeletal muscle (6) tissues. 1: normal testis, 8: H₂O.

(D) Expression of Piwil2 in different human cancer tissues. In most tumors, expression of Piwil2 was observed. Top: 1: colon cancer, 2: ovarian dysgerminoma, 3: malignant mixed mullerian tumor (MMMT) of the endometrium, 4: clear cell renal cell carcinoma, 5: gastrointestinal stromal tumor, 6: stromal sarcoma of endometrium, 7: adenocarcinoma of endometrium, 8: squamous cell carcinoma of pancreas, 9: normal blood, 10: H₂O, 11: adenocarcinoma of pancreas. Bottom: From tumor types with high expression level of Piwil2, tumor tissues from different patients were examined, 12-15: ovarian cancer, 16-19: prostate carcinoma, 20-26: mama carcinoma, 27-31: lymph node tumors, 32: H₂O.

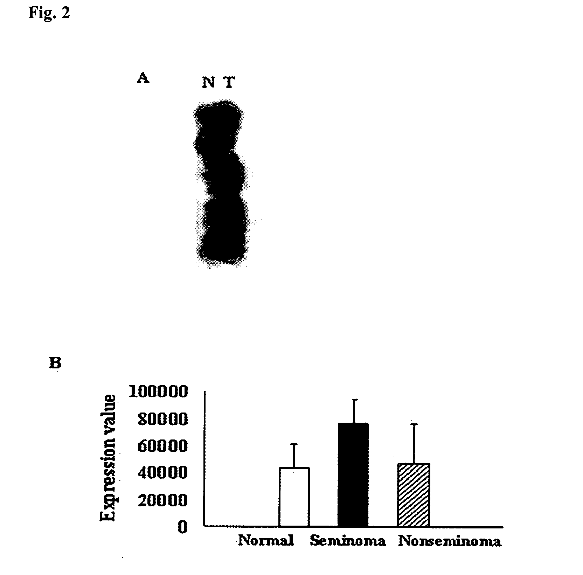

**Fig. 2.** Expression of piwil2 in testicular germ cell tumors. (A) Overexpression of Piwil2 was observed in 9 of 10 testicular seminomas (T) compared to normal testicular tissues (N). (B) Quantification of Piwil2 expression in seminomas and nonseminomatous tumors compared to normal testicular control tissues. Significant overexpression of Piwi12 was detected in seminomas (P<0.01) but not in testicular nonseminomatous tumors.

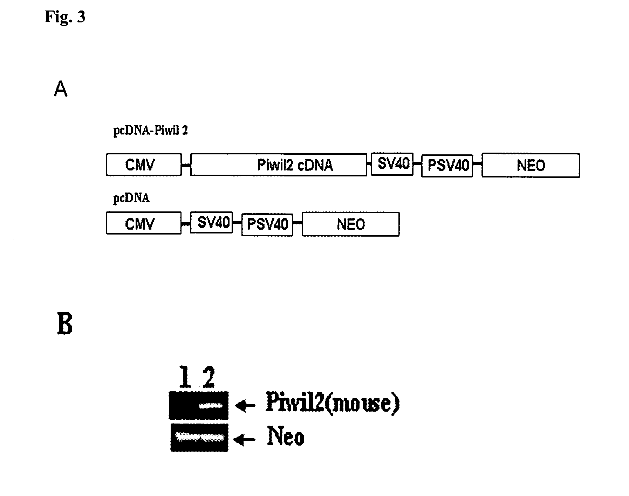

**Fig. 3.** Establishment of cell line with stable expression of Piwil2. (A) Schematic representation of fusion constructs used for transfection of NIH-3T3 cells, pcDNA-Piwil2 contains coding region of Piwi12 gene (cDNA) under control of cytomegalovirus (CMV) promoter and neomycin resistance gene (NEO) under control of SV40 promoter (PSV40). (B) RT-PCR analysis using RNA isolated from NIH-3T3 cell line (2) transfected with pcDNA-Piwil2 expressing mouse Piwil2 constitutively under control of cytomegalovirus promoter (NIH3T3-pcDNA-Piwil2) and NIH-3T3 cell line (1) transfected with a control vector without Piwil2 was used as control (NIH3T3-pcDNA). Expression of Piwil2 was detected using RT-PCR.

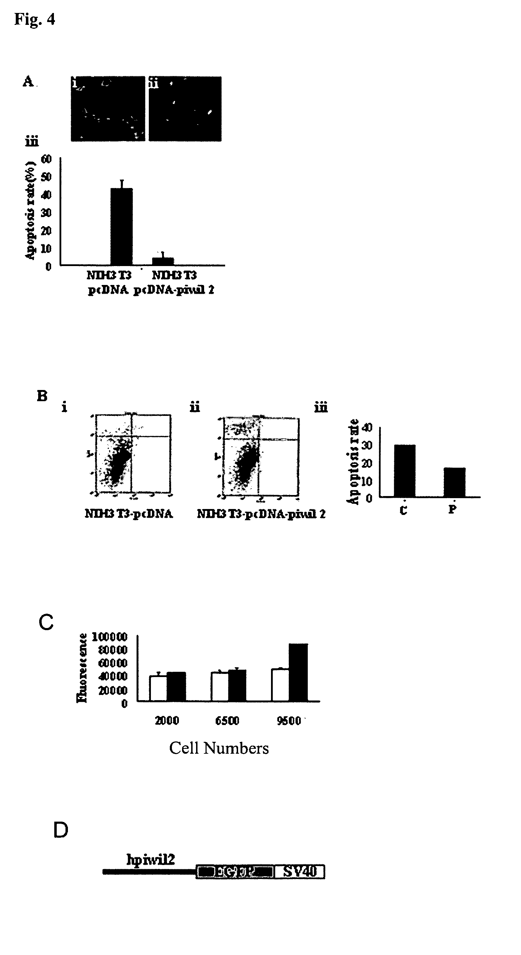

**Fig. 4**. Apoptosis and proliferation assay. (A) Apoptosis of control NIH3T3-pcDNA cell line (i) was compared with apoptosis of NIH-3T3 cell lines stably expressing Piwi12, NIH-3T3-pcDNA-Piwil2 (ii), revealed a drastically decrease of apoptosis by Piwil2 expression (p<0.01). (B) These results could be confirmed by apoptosis assay using FACS analysis. C: control cell line NIH3T3-pcDNA, P: NIH3T3-pcDNA-Piwil2. (C) In proliferation assay an increased cell proliferation was observed depending on the starting cell numbers. (D) Fusion gene harboring promoter of human Piwi12 gene (hpiwil2) and coding region of EGFP used for expression of EGFP in HeLa cells.

### EXAMPLES

### 1. MATERIALS AND METHODS

**1.1 Plasmid construction.** Plasmid pcDNA-Piwil2 was constructed using complete coding region (cDNA) of mouse Piwil2 gene obtained by PCR from a testis cDNA. The PCR product was cloned into pcDNA plasmid (BCCM/LMBP Plasmid collection, Gent, Belgium) containing promoter of cytomegalovirus (CMV). For hpiwil2-EGFP construct, 2 kb of flanking region of human Piwi12 gene was amplified and cloned into a plasmid vector containing coding region of EGFP gene and SV40 polyadenylation signals. Both plasmids contain neomycine resistance gene under control of SV40 early enhancer and promoter elements for positive selection. The final constructs were sequenced completely.

**1.2 Cell Culture.** Mouse NIH-3T3 fibroblasts were maintained in DMEM (PAN, Aidenbach, Germany) supplemented with 10% fetal calf serum, 1% glutamine (200mM), and 1% penicillin / streptomycin. HeLa cells were cultured in Dulbecco's modified Eagle's medium (DMEM) containing 10% heat-inactivated bovine serum (FBS), 2 mM L-glutamine, 100 U/ml penicillin, and 100 µg/ml streptomycin. Cells were cultured at 37 °C in a humidified atmosphere of 5% CO₂. Cells were plated at a density of 10⁶ cells/ml in 25-cm² culture flask and the monolayer was transfected with a freshly prepared liposome solution (55 °C) containing HEPES-NaCl and 1 µg/µL Clonfectin (BD Clontech, Palo Alto, CA). For the transfection, 4 µg of each construct DNA was mixed with 100 µl serum-free medium and 8 µg of Clonfectin and incubated for 30 min at RT. Cells were incubated in this solution for 4 h in a 5% CO₂ incubator. After washing in PBS (37 °C), expansion medium was added and cells were further cultivated for 3 days. HeLa cells were observed under a fluorescence microscope (Olympus BX60) after 48 h. Stable NIH-3T3 transfectants were selected by cultivating in medium containing 1 mg/ml of G-418 (PAN, Aidenbach, Germany) for 4 weeks. This cell line was designated as NIH3T3-pcDNA-Piwil2. As control, NIH-3T3 cells were transfected with pcDNA plasmid and the stable cell line was designated as NIH3T3-pcDNA.

**1.3 Northern Blot Hybridization.** Total RNA was isolated from NIH3T3-pcDNA cells and NIH3T3-pcDNA-Piwil2 cells using the RNeasy Mini kit (QIAGEN, Hilden, Germany) according to the manufacturer's instructions. Equal amounts of RNA were fractionated by formaldehyde agarose gel electrophoresis. Filters were hybridized with probes for Bcl-X_{L}, Akt, and Stat3, and after stripping, reprobed with human elongation factor-2 to ensure RNA concentrations and integrity.

**1.4 RNA Preparation and RT-PCR Analysis.** Total RNA was extracted from mouse and human tissues (normal and tumor) using TRIzol reagent (GIBCO-BRL) and from mouse and human cell lines (normal and tumor) using RNeasy Mini kit (QIAGEN, Hilden, Germany) according to the manufacturer's instructions. Single strand cDNAs were prepared from 5 µg RNA using a reverse transcriptase reaction (Invitrogen, Karlsruhe, Germany) and each PCR amplification was performed using Piwil2 specific primers (mouse: 5'-GCACAGTCCACGTGGTGGAAA-3' (SEQ ID NO:1), 5'-TCCATAGTCA-GGACCGGAGG G-3' (SEQ ID NO: 2), human: 5'-CAGGCAGAGGCCATGTATTT-3' (SEQ ID NO: 3), 5'-AACATGCCGACCTCATGCT-3' (SEQ ID NO: 4), and GAPDH primers used as a standard . The cycling conditions were as follows: 4 min at 94 °C, 5 cycles of 30 sec at 94 °C, 30 sec at 57-61 °C, 1 min at 72 °C, 30 cycles of 30 sec at 94 °C, 45 sec at 56 °C, 1 min at 72 °C, and 72 °C for 10 min.

Human total RNAs from colon, bone marrow, brain, small intestine, fetal brain, fetal liver, heart, kidney, spinal cord, lung, placenta, prostate, salivary gland, skeletal muscle, spleen, testis, stomach thyroid, trachea and uterus were purchased from BD Biosciences (BD Clontech, Palo Alto, CA). Mouse total RNAs were isolated from spleen, lung, heart, brain, kidney, skeletal muscle, ovary, liver, testis, breast and cerebellum. Furthermore, the following human and mouse tumor tissues were used for RNA isolation and RT-PCR analysis:
mouse: rhabomyosarcoma, medulloblastoma and breast tumors;
human: colon cancer, ovarian dysgerminoma, malignant mixed mullerian tumor (MMMT) of the endometrium, clear cell renal cell carcinoma, gastrointestinal stromal tumor, stromal sarcoma of endometrium, adenocarcinoma of endometrium, squamous cell carcinoma of pancreas, normal blood, adenocarcinoma of pancreas, ovarian cancer, prostate carcinoma, mama carcinoma and lymphatic gland tumors.

Following cell lines were used for isolation of RNAs and RT-PCR analysis:
mouse cell lines: transformed germ cells (GC-1 and GC-2), transformed Leydig cell line (MA-10), teratocarcinoma cell line F9, non-transformed spermatocyte (GC-4) and Sertoli cell (15-P1) line;
human cell lines: MDA-MB-237 (breast cancer), prostate cancer (PC-3, LNcAP, Du-145), HeLa (cervical cancer), Jurkat (T cell leukaemia), MCF-7 (breast adenocarcinoma), Daudi (Burkitt's lymphoma) and 2101EF (embryonal carcinoma), H12.1 (germ cell tumor).

**1.5 Apoptosis Assay.** Apoptosis was determined by two methods. The established cell lines (NIH3T3-pcDNA and NIH3T3-pcDNA-Piwi12) were fixed in 4% formaldehyde, processed for an apoptosis assay using the ApoAlert DNA fragmentation kit (BD Clontech, Palo Alto, CA) as described by the manufacturer, and apoptotic cells were observed and counted under a fluorescence microscope. Apoptotic rates were also analyzed by flow cytometry using FITC/propidium iodide (PI) staining. Staining was performed according to the manufacturer's instruction (BD Clontech, Palo Alto, CA). Fluorescence intensity measurements were done after excitation at 488 nm and detection at 520-530 nm for green fluorescence and at 665-685 nm for propidium iodide.

**1.6 Proliferation Assay.** NIH3T3-pcDNA and NIH3T3-pcDNA-Piwil2 cells were seeded at 2000, 6500, and 9500 cells/well in 96-well plates, respectively. Cells were cultivated in the microtiter plates for 2 h and 10 h. Quantification of cell proliferation was performed using Quantos cell proliferation assay kit (Stratagene, La Jolla, CA). Fluorescence of a DNA-dye complex from lysed cells was determined using a microtiter plate-reading fluorometer with filters appropriate for 355-nm excitation and 460-nm emission.

**1.7 Cancer Array Hybridization.** Human Cancer Profiling Array II (Clontech, Palo Alto, CA, USA) was hybridized with a human Piwi12 cDNA probe overnight and washed according to manufacturer's instruction. Quantitative evaluation of the array was performed by phosphor imager analysis using a molecular imager FX (BioRad, Hercules, CA, USA). For identification of differentially expressed genes, radioactive Cancer PathwayFinder Gene Array (GEArray Q series, SuperArray, Frederick, USA) was used.

Hybridization procedures were performed using a GEArray kit (SuperArray, Frederick, USA) as described by manufacturer.

**1.8 Histology and Immunohistochemistry.** NIH3T3-pcDNA and NIH3T3-pcDNA-Piwil2 cells were seeded on culture slides (FALCON, Le Pont De Claix, France) to 50 to 60% confluence. Cells were fixed in 4% paraformaldehyde solution, washed 3 times in PBS and then incubated with an anti-piwi12 antibody as primary antibodies in a humidifying chamber at 4°C overnight. The anti-piwi12 antibody is directed to the epitope DPVRPLFRG PTPVHP of the human piwi12 protein (The anti-Piwi12 antibody is generated using a synthetic peptide DPVRPLFRG PTPVHP in rabbit and recognizes specifically mouse and human Piwil2). After washing, culture slides were incubated with a 1:500 FITC- or Cy3-conjugated goat anti-rabbit IgG (Sigma-Aldrich, Munich, Germany) secondary antibody. Slides were washed 3 times in PBS and mounted in DAPI mounting solution (Vector Laboratories, Burlingame, CA). The cells were observed under a fluorescence microscope (Olympus BX60).

Human tissue samples (testis, testicular germ cell tumors, coccyx teratoma, ovarian dysgerminoma, ovarian teratoma, adenocarcinoma of colon, gastrointestinal stromal tumors, clear cell renal cell carcinoma, MMMT of endometrium, stromal sarcoma of endometrium, and adenocarcinoma of endometrium) were fixed in 4% paraformaldehyde in PBS, paraffin embedded and sectioned (3 µm). To remove paraffin from the slide, the paraffin-coated slide was washed 3 times with xylol for 3 min each, ethanol (100%, 96%, 90%, 80%, 70%, 50%) for 2 min each, dH₂O for 5 min and finally 3 times in PBS for 3 min, and then incubated with anti-Piwil2 antibody (directed to the epitope DPVRPLFRG PTPVHP of the human piwil2 protein) in a humidifying chamber at 4 °C overnight. After washing with PBS, primary antibody treated slides were incubated with 1:500 FITC-conjugated anti rabbit IgG (Sigma-Aldrich, Munich, Germany) for 2 hours, washed with PBS in 3 times for 3 min, stained with DAPI (Vector Laboratories, Burlingame, CA), and then observed under a fluorescence microscope (Olympus BX60).

### 2. RESULTS

### 2.1 Piwil2 is Expressed Specifically in Testis and in a Wide Variety of Tumors

Expression of mouse and human Piwil2 genes was analyzed using RT-PCR and primers specific for mouse and human Piwil2 genes. Testis specific expression was obtained in 20 normal adult human organs and 8 normal mouse organs (Fig. 1A). In RNAs isolated from different human and mouse cancer cell lines, a specific RT-PCR product was observed in most examined cell lines (8 of 10 human cell lines and 4 of 6 murine cell lines). No expression was detected in NIH-3T3 cells (Fig. 1B). To examine expression of Piwil2 in tumor tissues, RNAs were isolated from different mouse and human solid tumors and were subjected to RT-PCR analysis. As shown in Fig. 1C and 1D, expression of Piwil2 could be detected in most tumors (28 of 31 human tumors and 8 of 9 mouse tumors). Additionally, immunohistochemical analysis resulted in positive staining for Piwi12 in spermatogonia of normal seminiferous tubuli. No specific staining was detected in negative control of normal seminiferous tubuli of testis using secondary FITC-antibodies. Positive staining was also observed in most tumors either in dispersed (ovarian dysgerminoma, clear renal cell carcinoma, MMMT of endometrium, stromal sarcoma of endometrium, adenocarcinoma of endometrium) or in clonal form (teratoma of testis, testicular germ cell tumor, coccyx teratoma, adenocarcinoma of colon, gastrointestinal stromal tumors). These results indicate that Piwil2 is specifically expressed in spermatogonia of testis and ectopically in most tumor cell lines and tumor tissues.

Therefore, Piwil2 falls in category of cancer-testis antigens (CTAs). Specific genes are expressed in the testis and in tumors of varying histological origin. In testis, CTAs are exclusively present in cells of the germ cell lineage, though there is a lot of variation in the expression profile during different stages of germ cell development (Zendman et al., J Cell Physiol. 2003; 194:272-88). The tissue expression pattern indicates that these antigens are targets for active specific immunotherapy.

### 2.2 Piwil2 is Overexpressed in Testicular Germ Cell Tumors

To examine expression of Piwil2 in human testicular germ cell tumors, a human testicular cancer profiling array with RNAs from seminomas, nonseminomatous tumors and surrounding normal testicular tissue, was hybridized with a Piwil2 specific probe. As shown in Fig. 2A and Fig. 2B, an overexpression of Piwil2 was observed in seminomas. No enhanced expression of Piwil2 was detected in testicular nonseminomatous tumors (Fig. 2B). This expression pattern mimics expression pattern of Piwill in testicular germ cell tumors. In normal human testes, Piwill (hiwi) is specifically expressed in germ cells, with its expression detectable in spermatocytes and round spermatids during spermatogenesis (Qiao et al., supra). Enhanced expression of Piwill was found in 12 out of 19 sampled testicular seminomas originating from embryonic germ cells with retention of germ cell phenotype. In contrast, no enhanced expression was detected in 10 nonseminomatous testicular tumors that originate from the same precursor cells as seminomas yet have lost their germ cell characteristics (Qiao et al., supra). These results indicate a possible synergistic effect of piwi genes on initiation and progression of testicular germ cell tumors.

### 2.3 Identification of Piwil2 Downstream Targets

To search for potential molecular downstream targets of Piwil2, we established NIH-3T3 cell lines stably expressing Piwi12 using a fusion gene harboring coding region of Piwil2 under control of CMV promoter (Fig. 3A). Expression of Piwil2 in the stable cell line NIH3T3-pcDNA-Piwil2 was examined on RNA (Fig. 3B) and also on protein level. It was demonstrated that Piwil2 is transcribed and translated properly in NIH3T3-pcDNA-Piwil2 cell line. Expression profiles of NIH-3T3 cells expressing stably full length Piwi12 was compared with control cell line NIH3T3-pcDNA using a Cancer Pathway Finder Array. This analysis revealed activation of genes related to cell growth, adhesion and apoptosis (Table 1).

**Table 1: Differential gene expression in NIH-3T3 cells expressing Piwil2 compared to NIH-3T3 control cells. Overexpression of apoptosis, growth and adhesion related genes was obtained.**

| **Symbol** | **Gene name** | **Expression level in NIH3T3-pcDNA-Piwil2 Cells** |
|---|---|---|
| **Agpt** | **angiopoietin1** | **▼** |
| **Bc121** | **Bcl-x** | **▲** |
| **Cflar** | **Casper(CASP8 and FADD-like apoptosis regulator** | **▲** |
| **Catnb** | **Catenin beta** | **▲** |
| **Ccnd1** | **Cyclin D1** | **▲** |
| **Cd44** | **CD44 antigen** | **▲** |
| **Grb2** | **Grb2** | **▲** |
| **Itga6** | **Integrin alpha 6** | **▲** |
| **Itgb1** | **Integrin betal(fibronectin receptor beta):CD29** | **▲** |
| **Mmp2** | **Matrix metalloproteinase2 :Gelatinase A** | **▲** |
| **Thbs1** | **thrombospondin1** | **▼** |

An activation of Bcl-X expression was obtained specifically in NIH3T3-pcDNA-Piwil2 cell line. Northern blot analysis of RNA isolated from control NIH-3T3, pcDNA-Piwil2, and NIH-3T3 stably expressed Piwil2, NIH3T3-pcDNA-Piwil2, using probes specific for Akt, Bel-X_{L}, cyclin D1 and Stat3. An activation of Stat3/Bcl-X_{L} and signalling pathway and an enhancement of cyclin D1expression were observed.

An activation of Bcl-x was observed in cells expressing Piwil2. Bcl-X belongs to bcl-2 gene family. Members of the bcl-2 family of genes serve as regulators of cell death, either promoting (Bax, Bak, Bok, Bik, Blk, Hrk, Bad, Bid, Diva, and EGL-1) or inhibiting it (Bcl-2, Bcl-X, Bcl-w, Mcl-1, and CED-9). To examine whether expression of Bcl-x is regulated directly by Piwi12 or indirectly by upstream regulatory factors of Bcl-X, expression of the signal transducers and activators of transcription 3 (Stat3) and serine/threonine kinase Akt, two upstream regulatory factors of Bcl-X, were examined. Whereas no detectable change was observed in expression level of Akt, an activation of Stat3 was detected in cells stably expressing Piwil2. This indicates the role of Piwil2 as upstream regulatory factor of Stat3. An overexpression of cyclin D1, a downstream gene of Stat3, was also observed. The signal transducers and activators of transcription (STAT) factors function as downstream effectors of cytokine and growth factor receptor signaling. In mouse, STAT3 activation is required and sufficient to maintain the undifferentiated state of ES cells (Matsuda et al., EMBO J., 1999; 18:4261-9; Niwa et al., Genes Dev. 1998; 12:2048-60). Compared with normal cells and tissues, constitutively activated STATs have been detected in a wide variety of human cancer cell lines and primary tumors (Buettner et al., Cancer Res., 2002; 8:945-54). STATs are activated by tyrosine phosphorylation, which is normally a transient and tightly regulated process. In tumor cells, constitutive activation of STATs is linked to persistent activity of tyrosine kinases, including Src, epidermal growth factor receptor, Janus kinases, Bcr-Abl, and many others. Such oncogenic tyrosine kinases are often activated as a consequence of permanent ligand/receptor engagement in autocrine or paracrine cytokine and growth factor signaling or represent autonomous constitutively active enzymes as a result of genetic alterations found in tumor but not in normal cells. Persistent signaling of specific STATs, in particular Stat3 and Stat5, has been demonstrated to directly contribute to oncogenesis by stimulating cell proliferation and preventing apoptosis. STATs participate in oncogenesis through up-regulation of genes encoding apoptosis inhibitors and cell cycle regulators such as Bcl-x(L), Mcl-1, cyclins D1/D2, and c-Myc. These results demonstrate that Piwi12 independently targets two important cellular signalling pathways, Stat3/Bc1-X and Stat3/cyclin D1 and therefore can act as an oncogenic factor in tumorigenesis of different tissues.

### 2.4 Piwil2 Inhibits Apoptosis and Stimulates Cell Proliferation.

To study the causal effect of Piwil2 expression on apoptosis and cell proliferation, NIH-3T3 cells stably expressing Piwil2 (NIH3T3-pcDNA-Piwil2) were compared to control cells NIH3T3-pcDNA. Using two different techniques, microscopically and cytometrically, a significant decrease of apoptosis was observed in NIH-3T3 cells expressing Piwil2 (Fig. 4A and 4B). Using a combined apoptosis assay and Piwil2 immunostaining, it was demonstrated that apoptotic cells do not express Piwil2 and cells expressing Piwil2 are not apoptotic. These results are in agreement with the results obtained in Piwi12 knockout mice. In Piwi12 null mice an increase of apoptotic germ cells was detected (Kuramochi-Miyagawa et al, supra).

In proliferation assay, an increased proliferative activity of NIH-3T3 cells expressing Piwil2 was obtained (Fig. 4C). Increased proliferation was observed if the number of starting cells was higher that 9000 and not detectable if the starting cell number was low (for example 2000 and 6500 starting cells in Fig. 4C), an indication for cell-cell interaction in Piwil2-mediated cell proliferation.

To examine the correlation between Piwi12 expression and cell cycle, 2 kb of human Piwi12 flanking region was linked to EGFP (Fig. 4D). This fusion construct was transfected into the HeLa cells and expression of EGFP was observed after 48 h. Expression of EGFP was always observed in proliferative active cells with condensed metaphase chromosomes. In NIH3T3-pcDNA-Piwil2, expression of Piwil2 was also observed in proliferative active cells.

These results demonstrate that Piwil2 inhibits apoptosis and stimulate proliferation through activation of Stat3/Bcl-x and enhancement of Stat3/cyclin D1 signaling pathways. Inhibition of constitutively signaling pathways by repression of Piwil2 expression can inhibit tumor cell growth in vitro and in vivo and provides a novel means for therapeutic intervention in human cancer. Furthermore, specific expression of Piwil2 in testis and its aberrant expression in various tumors make this molecule an attractive cancer prognostic and diagnostic marker.

## Claims

1. A method of diagnosing cancer other than testicular cancer comprising the step of:
(a) determining the amount of piwi-like 2 (piwil2) in a subject-derived sample,
wherein an increased amount of piwil2 in the sample in comparison with a control value is indicative for cancer.

2. The method of claim 1, wherein the subject is a mammal, preferably a human.

3. The method of claim 1 or 2, wherein the sample is a blood sample, tissue sample, urine sample, saliva sample, a serum sample or a plasma sample.

4. The method of any of claims 1 to 3, wherein the amount of piwi12 is determined by measuring the amount of mRNA encoding the piwil2 protein, the amount of piwil2 protein and/or the amount of anti-piwi12 antibody in the sample.

5. The method of any of claims 1 to 4, wherein the amount of mRNAs is determined by a hybridization method or a nucleic acid amplification method, in particular by using a piwil2-based primer, especially a primer as defined in SEQ ID NO:1, SEQ ID NO:2, SEQ ID NO:3 or SEQ ID NO:4 or a homologue thereof.

6. The method of any of claims 1 to 4, wherein the amount of piwil2 protein or of anti-piwi12 antibody is determined by using binding proteins capable of binding specifically to said protein, in particular an antibody or antibody fragment, especially an anti-piwil2 antibody recognizing an epitope of the piwil2 protein showing no homology to other piwi proteins, particularly wherein the epitope is located N-terminally, more particularly wherein the antibody recognizes epitope "DPVRPLFRG PTPVHP" of the piwi12 protein or a homologue thereof.

7. The method of any of claims 1 to 6 for use in early detection, to monitor or assess a cancer's response to treatment and/or to check for recurrence.

8. Use of an indicator of piwil2 for the diagnosis of cancer other than testicular cancer.

9. The use of claim 8, wherein the indicator is
(i) a nucleic acid, in particular a primer pair and/or a probe, particularly comprising a piwil2-based primer, especially a primer as defined SEQ ID NO:1, SEQ ID NO:2, SEQ ID NO:3 or SEQ ID NO:4 or a homologue thereof; or
(ii) an antibody or antibody fragment, especially an anti-piwil2 antibody recognizing an epitope of the piwil2 protein showing no homology to other piwi proteins, particularly wherein the epitope is located N-terminally, more particularly wherein the antibody recognizes epitope "DPVRPLFRG PTPVHP" of the piwil2 protein or a homologue thereof.

10. A kit suitable for diagnosing a cancer other than testicular cancer, comprising
(i) a primer pair and/or a probe suitable for determining the amount of mRNA encoding piwil2, in particular comprising a piwil2-based primer, especially a primer as defined in SEQ ID NO: 1, SEQ ID NO:2, SEQ ID NO:3 or SEQ ID NO:4 or a homologue thereof; and/or
(ii) an antibody or antibody fragment, especially an anti-piwil2 antibody recognizing an epitope of the piwi12 protein showing no homology to other piwi proteins, particularly wherein the epitope is located N-terminally, more particularly wherein the antibody recognizes epitope "DPVRPLFRG PTPVHP" of the piwil2 protein or a homologue thereof.

11. A method of screening for an inhibitor of piwi12 for use in the treatment or prevention of cancer comprising the steps of:
(a) exposing a test cell expressing the piwil2 protein to a test compound; and
(b) comparing the rate of apoptosis or the rate of proliferation of the test cell with that of a control cell,
wherein an increased rate of apoptosis or an decreased rate of proliferation of the test cell in comparison with that of the control cell is indicative for an inhibitor of piwil2.

12. The method of claim 11, wherein the test compound is provided in the form of a chemical compound library and/or wherein the method is a method of high-through put screening.

13. The method of claim 11 or 12 further comprising the step of incorporating the inhibitor of piwil2 into a therapeutic or prophylactic composition.

14. Use of an anti-piwil2 antibody, an anti-piwil2 antibody fragment, a microprotein, an antisense polynucleotide or small interfering RNA of a polynucleotide encoding piwil2 for the manufacture of a medicament for treating or preventing cancer.

15. The method of any of claims 1 to 7 or 11 to 13, the use of any of claims 8, 9 or 14, or the kit of claim 10, wherein the cancer is breast tumor, cervical cancer, leukemia, lymphoma, medullablastoma, rhabdomyosarcoma, colon cancer, ovarian cancer, endometrial cancer, renal cancer, gastrointestinal cancer, pancreas cancer, blood cancer, prostate cancer or lymph node tumor.


SEQUENCE LISTING

<110> Georg-August-Universität Göttingen

<120> Method of diagnosing cancer

<130> K63083EP

<160> 5

<170> PatentIn version 3.1

<210> 1
<211> 21
<212> DNA
<213> artificial sequence

<220>
<223> primer

<400> 1
gcacagtcca cgtggtggaa a 21

<210> 2
<211> 21
<212> DNA
<213> artificial sequence

<220>
<223> primer

<400> 2
tccatagtca ggaccggagg g 21

<210> 3
<211> 20
<212> DNA
<213> artificial sequence

<220>
<223> primer

<400> 3
caggcagagg ccatgtattt 20

<210> 4
<211> 19
<212> DNA
<213> artificial sequence

<220>
<223> primer

<400> 4
aacatgccga cctcatgct 19

<210> 5
<211> 15
<212> PRT

<213>  artificial sequence

<220>
<223>  epitope of piwil2 protein

<400>  5

Asp Pro Val Arg Pro Leu Phe Arg Gly Pro Thr Pro Val His Pro
1               5                   10                  15